# EUROPEAN PATENT APPLICATION

(11) **EP 2 952 135 A1**
(43) Date of publication of application: **09.12.2015**
(21) Application number: 14853152.8
(22) Date of filing: 21.08.2014
(51) Int. Cl.: A61B 5/01

(54) **TEMPERATURE MEASUREMENT DEVICE AND TEMPERATURE MEASUREMENT METHOD**

(30) Priority: 31.03.2014 CN 201410127762
(71) Applicant: BOE Technology Group Co., Ltd., Beijing 100015 (CN)
(72) Inventor: ZHANG, Kailiang, Beijing 100176 (CN)
(74) Representative: Cohausz & Florack
(86) International application number: PCT/CN2014/084912
(87) International publication number: WO 2015/149470

(57) **Abstract**

The present invention provides a temperature measurement device and a temperature measurement method. The temperature measurement device includes a first sensor, used for collecting a body surface temperature of an organism; a second sensor, used for collecting an ambient temperature; and a processing unit, which is electrically connected with the first sensor and the second sensor respectively, and is used for performing data processing on the body surface temperature according to the ambient temperature to obtain an actual body temperature of the organism. Due to the arrangement of the first sensor, the second sensor and the processing unit, the temperature measurement device can perform data processing on the collected body surface temperature according to the ambient temperature to make the obtained actual body temperature of the organism more accurate, and further to facilitate accurately judging the health status of the organism.

## Description

### Field of the Invention

The present invention relates to the field of temperature measurement, and particularly relates to a temperature measurement device and a temperature measurement method.

### Background of the Invention

Wearable electronic products are widely used due to ease of use. It is an important application of the wearable electronic products that some physical sign parameters of a human body are obtained by a wearable electronic product, and then the health status of the human body is judged based on the obtained physical sign parameters.

Body temperature is an important physical sign parameter of the human body, and a lot of physical abnormities can be reflected by temperature abnormity, so temperature measurement is particularly important for health monitoring of the human body, and the accuracy of the temperature measurement has a significant influence on the judgment of the health status. The general temperature detection is axillary temperature detection, but body temperature at other body surface position, such as the wrist, the neck or the like, is also detected sometimes. However, the body temperature at the body surface position such as the wrist, the neck or the like is very likely to be influenced by the ambient temperature.

At present, a watch capable of measuring the body temperature at the wrist appears on the market, but since the body temperature at the wrist is often affected by the ambient temperature and thus deviates from the actual body temperature of the human body, the measured temperature is not the true body temperature of the human body generally. In this case, a user needs to judge whether his own body temperature is normal according to the previously accumulated measurement experience, and since this judgment is influenced by subjective factors more or less, grasp of the actual body temperature of the human body is not accurate enough.

### Summary of the Invention

An object of the present invention is to provide a temperature measurement device and a temperature measurement method, in view of the above-mentioned technical problems in the prior art. The temperature measurement device can perform data processing on the collected body surface temperature according to an ambient temperature to make the obtained actual body temperature of an organism more accurate, and further to accurately judge the health status of the organism according to the obtained actual body temperature.

According to one aspect of the present invention, there is provided a temperature measurement device, including a first sensor, used for collecting a body surface temperature of an organism; a second sensor, used for collecting an ambient temperature; and a processing unit, which is electrically connected with the first sensor and the second sensor, respectively, and is used for performing data processing on the body surface temperature according to the ambient temperature to obtain an actual body temperature of the organism.

Preferably, the processing unit includes a compensation module, which is used for compensating the body surface temperature with a compensation value, the compensation value is a deviation of the ambient temperature from an ambient normal temperature, and the ambient normal temperature is a standard temperature of an environment where the organism is located.

Preferably, the processing unit may further include an analog/digital conversion module, which is electrically connected with the first sensor and the second sensor, respectively and is used for respectively converting the body surface temperature and the ambient temperature from analog quantities into digital quantities and transmitting the digital quantities of the body surface temperature and the ambient temperature to the compensation module.

Preferably, the processing unit may further include an averaging module, which is electrically connected with the compensation module and is used for averaging two or more compensated body surface temperatures to obtain an average value serving as the actual body temperature of the organism.

Preferably, the processing unit may further include a correction module, which is electrically connected with the compensation module and is used for correcting the compensated body surface temperature with a correction value, and the correction value is an empirical value obtained through a plurality of tests and represents a deviation of an actual body surface temperature from a true body temperature of the organism.

Preferably, the processing unit may further include an averaging module, which is electrically connected with the correction module and is used for averaging two or more compensated and corrected body surface temperatures to obtain an average value serving as the actual body temperature of the organism.

Preferably, the temperature measurement device may further include a storage unit, which is electrically connected with the processing unit, and is used for storing data in data processing.

Preferably, the temperature measurement device may further include an annular belt, which is worn on the organism, the first sensor is arranged at an inner side of the annular belt facing a body surface of the organism, and the second sensor is arranged at an outer side of the annular belt away from the body surface of the organism.

Preferably, a distance from the first sensor to the body surface of the organism may be in the range of 0 to 2mm, and a distance from the second sensor to the body surface of the organism may be in the range of 5 mm to 8mm.

Preferably, the body surface temperature may include a body surface temperature at wrist of the organism, a body surface temperature at neck of the organism or a body surface temperature at ankle of the organism.

Preferably, the processing unit may include a single chip microcomputer, a DSP or an FPGA, and the first sensor and the second sensor respectively transmit the body surface temperature and the ambient temperature to the processing unit through a communication protocol.

According to another aspect of the present invention, there is provided a temperature measurement method, including: step S1: collecting a body surface temperature of an organism, and collecting an ambient temperature; and step S2: performing data processing on the body surface temperature according to the ambient temperature to obtain an actual body temperature of the organism.

Preferably, step S2 of performing data processing on the body surface temperature according to the ambient temperature to obtain an actual body temperature of the organism includes: step S21: compensating the body surface temperature with a compensation value, which is a deviation of the ambient temperature from an ambient normal temperature, wherein the ambient normal temperature is a standard temperature of an environment where the organism is located; and step S22: correcting the compensated body surface temperature with a correction value, which is an empirical value obtained through a plurality of tests and represents a deviation of an actual body surface temperature from a true body temperature of the organism.

Preferably, step S2 of performing data processing on the body surface temperature according to the ambient temperature to obtain an actual body temperature of the organism further includes: step S23: repeatedly performing the above-mentioned step S1, step S21 and step S22 for twice or more times, and then averaging the obtained two or more compensated and corrected body surface temperatures.

Preferably, before step S21 of compensating the body surface temperature, the method further includes: converting the body surface temperature and the ambient temperature from analog quantities into digital quantities, respectively.

The present invention has the beneficial effects as follows: in the temperature measurement device provided by the present invention, with the first sensor and the second sensor, the temperature measurement device can collect the body surface temperature of the organism and the ambient temperature, and with the processing unit, the temperature measurement device can perform data processing on the collected body surface temperature according to the ambient temperature to obtain the actual body temperature of the organism. By providing the processing unit, the obtained actual body temperature of the organism is more accurate, thus facilitating accurately judging the health status of the organism according to the actual body temperature. In addition, the temperature measurement device is economical in consumables, easy to implement and convenient to use.

According to the temperature measurement method provided by the present invention, by collecting the body surface temperature of the organism and the ambient temperature, and performing data processing on the collected body surface temperature according to the ambient temperature, the actual body temperature of the organism can be obtained quickly and accurately so as to judge the health status of the organism conveniently and accurately.

### Brief Description of the Drawings

Fig.1 is a schematic block diagram of a temperature measurement device provided by an embodiment of the present invention;
Fig.2 is a flowchart of a temperature measurement method provided by an embodiment of the present invention; and
Fig.3 is a flowchart of a specific temperature measurement process of the temperature measurement method as shown in Fig.2.

### Detailed Description of the Embodiments

To make those skilled in the art better understand the technical solutions of the present invention, a temperature measurement device and a temperature measurement method provided by the present invention will be further described in detail below in combination with the accompanying drawings and specific implementations.

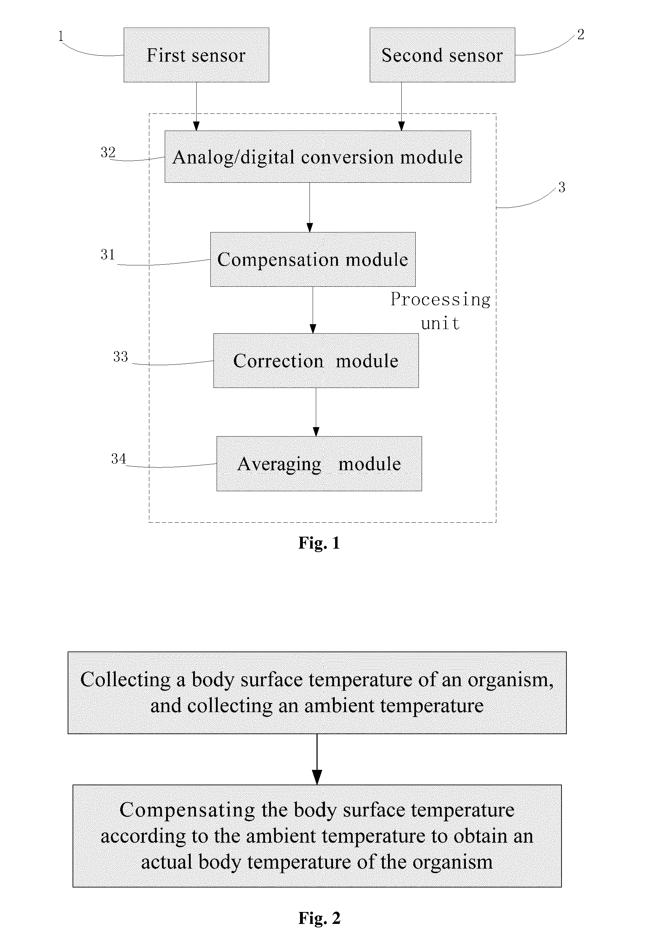

An embodiment of the present invention provides a temperature measurement device, as shown in Fig.1, including a first sensor 1, which is used for collecting a body surface temperature of an organism; a second sensor 2, which is used for collecting ambient temperature; and a processing unit 3, which is electrically connected with the first sensor 1 and the second sensor 2 respectively, and is used for performing data processing on the collected body surface temperature according to the ambient temperature to obtain an actual body temperature of the organism.

Here, the ambient temperature is a temperature of the environment where the organism is located. The first sensor 1 and the second sensor 2 may be sensors adopting any working principle, as long as they can collect the body surface temperature of the organism or the ambient temperature.

In this embodiment, the processing unit 3 includes a compensation module 31, which is used for compensating the body surface temperature with a compensation value, which may be a deviation of the ambient temperature from an ambient normal temperature, and the ambient normal temperature is a standard temperature of the environment where the organism is located.

For example, the body surface temperature is set as Ta, the ambient temperature is set as Tb, and the ambient normal temperature is assumed to be a room temperature of 25°C. Since the body surface temperature may vary relatively to the ambient normal temperature under the influence of the ambient temperature, the body surface temperature needs to be compensated according to the ambient temperature. The compensation value may be Tb-25°C, then the compensated body surface temperature is Ta-(Tb-25°C), which is closer to the true body temperature. The compensation module 31 can compensate for the influence of the ambient temperature on the body surface temperature, so that the actual body temperature of the organism obtained via the temperature measurement device is more accurate.

The temperature measurement device may further include a storage unit, which is electrically connected with the processing unit, and is used for storing data in the data processing.

It needs to be noted that, for example, the above-mentioned ambient normal temperature may be a temperature in a room where people are located and is generally about 20°C, and since people usually measure the temperature under such ambient normal temperature, the body temperature measured at such temperature has a better reference value. The ambient normal temperature may be stored in the storage unit of the temperature measurement device in advance. For example, the ambient normal temperature stored in the storage unit may be 25°C ; of course, for accuracy and humanization, the user can manually set the ambient normal temperature according to the environment where the temperature measurement device is located and store a numerical value of the set ambient normal temperature in the storage unit for use in subsequent data processing.

In this embodiment, the processing unit 3 may further include an analog/digital conversion module 32, which is electrically connected with the first sensor 1 and the second sensor 2, respectively and is used for respectively converting the body surface temperature and the ambient temperature from analog quantities into digital quantities and transmitting the digital quantities of the body surface temperature and the ambient temperature to the compensation module 31. By providing the analog/digital conversion module 32, the compensation module 31 directly compensates the body surface temperature and the ambient temperature which are digital quantities, thus facilitating the compensation calculation of the actual body temperature of the organism and ensuring a more accurate and quicker compensation calculation of the actual body temperature.

In this embodiment, the processing unit 3 may further include a correction module 33, which is electrically connected with the compensation module 31 and is used for correcting the compensated body surface temperature with a correction value, and the correction value may an empirical value obtained through multiple measurements and represents the deviation of the actual body surface temperature (in this embodiment, the actual body surface temperature may be represented by the compensated body surface temperature) from the true body temperature of the organism.

For example, it is learned from several tests that, the actual body surface temperature at the wrist of the human body is 4°C lower than the true body temperature of the human body (the true body temperature can be generally represented by the body temperature measured at the oral cavity, the rectum or the armpit), therefore the body surface temperature obtained by correcting the compensated body surface temperature (here, the actual body surface temperature at the wrist) is Ta-(Tb-25°C) +4°C, and the compensated and corrected body surface temperature is closer to the true body temperature than the simply compensated body surface temperature. The correction module 33 can correct the deviations of actual body surface temperatures at different parts of the organism from the true body temperature of the organism, so that the actual body temperature of the organism obtained via the temperature measurement device is more accurate (i.e., closer to the true body temperature of the organism).

It needs to be noted that, since the body surface temperature includes the body surface temperature at different parts such as the wrist, the neck or the ankle or the like of the organism, and there are certain deviations between the actual body surface temperatures at different parts of the organism and the true body temperature of the organism, in order to obtain more accurate actual body temperature of the organism, the body surface temperature needs to be corrected. The correction value used for correcting the body surface temperature can be stored in the storage unit in advance, and for example, the correction value stored in a measurement device for the wrist may be 4°C. Of course, for accuracy and humanization, the user may manually set the correction value according to the environment where the measurement device is located or the part of the organism for measurement and store the set correction value in the storage unit for use in subsequent data processing.

In this embodiment, the processing unit 3 may further include an averaging module 34, which is electrically connected with the correction module 33 and is used for averaging two or more compensated and corrected body surface temperatures to obtain an average value, which is taken as the actual body temperature of the organism.

For example, N (N is an integer larger than 1) compensated and corrected body surface temperatures (for example, the body surface temperatures at the wrist) are averaged to obtain the actual body temperature of the human body: { ( Tal-( Tb1-25°C ) +4°C ) + ( Ta2- ( Tb2-25 °C ) +4°C ) +......+ ( TaN- ( TbN-25 °C ) +4°C ) } /N, wherein the larger the value of N is, the more accurate the calculation result is. The averaging module 34 can obtain the average value of multiple compensated and corrected body surface temperatures, and the averaged body surface temperature is generally closer to the true body temperature of the organism, and thus the health status of the organism can be accurately judged according to the obtained actual body temperature (i.e., the averaged body surface temperature).

To facilitate the user to obtain measurement data, the above-mentioned measurement device may further include a display unit, for example, a liquid crystal display device, and in this way, the user can visually obtain the measurement data. Of course, the above-mentioned measurement device may further include an audio unit used for broadcasting final measurement data, and in this way, the user can aurally obtain the measurement data. The specific manner of obtaining the measurement data will not be repeated redundantly herein.

In this embodiment, the temperature measurement device may further include an annular belt (not shown in Fig.1), which is worn on the organism, the first sensor 1 is arranged at an inner side of the annular belt facing the body surface of the organism, and the second sensor 2 is arranged at an outer side of the annular belt away from the body surface of the organism. Due to the arrangement of the annular belt, the temperature measurement device is convenient to carry, thus facilitating real-time measurement of the actual body temperature of the organism. Here, a distance from the first sensor 1 to the body surface of the organism preferably is in the range of 0 to 2mm, and a distance from the second sensor 2 to the body surface of the organism preferably is in the range of 5 mm to 8mm. With such arrangement, it is convenient for the first sensor 1 and the second sensor 2 to respectively perform nearest measurement on the body surface temperature of the organism and the ambient temperature of the environment where the organism is located, so as to compensate and correct the body surface temperature of the organism more accurately.

It needs to be noted that, the organism mentioned in this embodiment generally refers to a human body or an animal body.

In this embodiment, the processing unit 3 may include a single chip microcomputer, a DSP or an FPGA, the first sensor 1 and the second sensor 2 may respectively transmit the body surface temperature and the ambient temperature to the processing unit 3 through a communication protocol. For example, the communication protocol may be an SPI communication protocol, an IIC communication protocol or the like, and by means of the SPI communication protocol or the IIC communication protocol, the communication between the first and second sensors 1 and 2 and the processing unit 3 is more convenient and quicker, which facilitates the processing unit 3 to calculate and process the body surface temperature faster and more accurately, so as to obtain the actual body temperature of the organism faster.

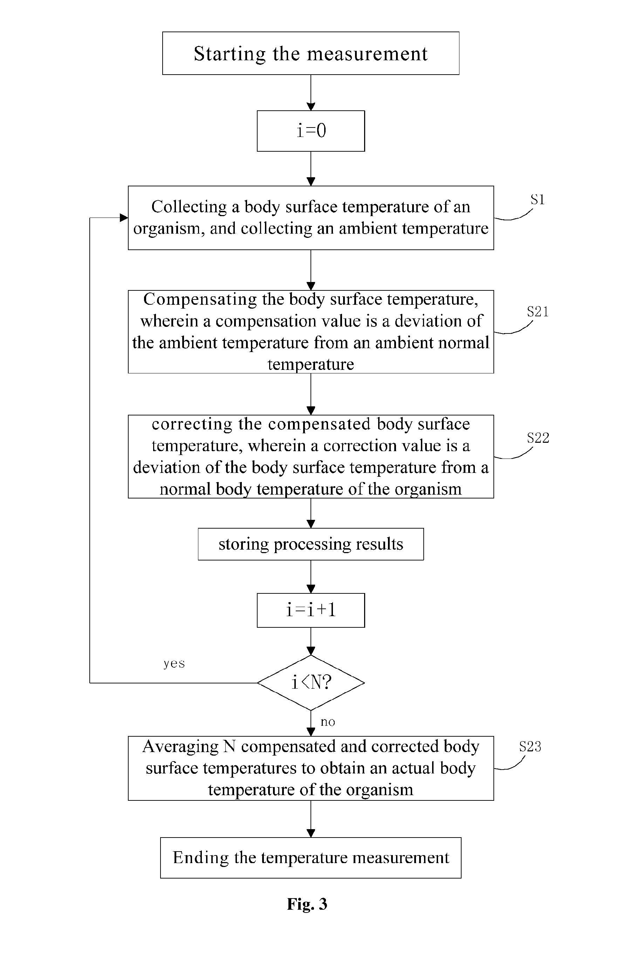

Based on the above-mentioned temperature measurement device, an embodiment of the present invention further provides a temperature measurement method, and as shown in Fig.2 and Fig.3, the method includes: step S1: collecting a body surface temperature of an organism and an ambient temperature, wherein, the ambient temperature is a temperature of an environment where the organism is located; and step S2: performing data processing on the body surface temperature according to the ambient temperature to obtain an actual body temperature of the organism.

The step S2 includes: step S21: compensating the body surface temperature with a compensation value, which is a deviation of the ambient temperature from an ambient normal temperature, wherein the ambient normal temperature is a standard temperature of the environment where the organism is located; and step S22: correcting the compensated body surface temperature with a correction value, wherein the correction value is a deviation of the compensated body surface temperature from the true body temperature of the organism, and the correction value is an empirical value obtained through multiple tests.

To make the actual body temperature obtained by the temperature measurement method provided in this embodiment be more accurate, the step S2 may further include step S23: repeatedly performing the above-mentioned step S1, step S21 and step S22 for N times (N is an integer larger than 1), to obtain N compensated and corrected body surface temperatures, and then averaging the obtained N compensated and corrected body surface temperatures.

For example, N (N is an integer larger than 1) body surface temperatures are compensated, corrected and averaged, and the specific processing process of the measured body surface temperature is as follows: as shown in Fig. 3, once the measurement is started, firstly, an initial counting value i of a counter is set as 0, sequentially performing step S1, step S21 and step S22, storing the processing results (i.e., the compensated and corrected body surface temperatures), and accumulatively performing counting for one time in the counter (adding 1 to the counting value for each loop processing, namely, i=i+1); then judging whether the counting value i is smaller than N, if not, performing step S23: averaging two or more (i.e., N) compensated and corrected body surface temperatures to obtain the actual body temperature of the organism, and ending the temperature measurement; or otherwise, returning to step S1 to perform a next loop processing.

Through the above-mentioned steps, the temperature measurement method provided by the present invention can be used for obtaining the actual body temperature of the organism quickly and accurately, which is conducive to accurately judging the health status of the organism.

The embodiments of the present invention have the beneficial effects as follows: according to the temperature measurement device in the embodiment, with the first sensor and the second sensor, the temperature measurement device can collect the body surface temperature of the organism and the ambient temperature, respectively, by providing the compensation module, the correction module and the averaging module, the temperature measurement device can compensate the body surface temperature according to the ambient temperature, correct the body surface temperature according to the deviations of the body surface temperatures at different parts of the organism from the true body temperature of the organism and finally average multiple compensated and corrected body surface temperatures to obtain the actual body temperature of the organism, and this makes the obtained actual body temperature of the organism more accurate, which is conducive to accurately judging the health status of the organism.

It should be understood that, based on the above-mentioned embodiments, various variations can be made in the present invention. For example, the processing unit of the temperature measurement device provided by the present invention may include no analog/digital conversion module. For example, under the condition that the body surface temperature and the ambient temperature collected by the first sensor and the second sensor are digital quantities, the processing unit of the temperature measurement device does not need to be provided with the analog/digital conversion module. In this case, since the other structures of the temperature measurement device are the same as those in the above embodiment, they will not be repeated redundantly herein.

As another example, in order to simplify the measurement device and make the measurement device simpler, the processing unit of the temperature measurement device provided by the present invention may include no correction module or averaging module. When the processing unit of the temperature measurement device includes the averaging module but includes no correction module, the averaging module is electrically connected with the compensation module and is used for averaging two or more compensated body surface temperatures to obtain an average value, which is taken as the actual body temperature of the organism. In this case, since the other structures of the temperature measurement device are the same as those in the above embodiment, they will not be repeated redundantly herein.

When the processing unit includes no averaging module, the actual body temperature obtained by the temperature measurement device is the body surface temperature of the organism obtained after compensation and correction for once in the current environment. When the processing unit includes no correction module, the actual body temperature obtained by the temperature measurement device is the compensated and averaged body surface temperature of the organism in the current environment.

The present invention has the beneficial effects as follows: in the temperature measurement device provided by the present invention, with the first sensor and the second sensor, the temperature measurement device can collect the body surface temperature of the organism and the ambient temperature, respectively, and with the processing unit, the temperature measurement device can perform data processing on the body surface temperature according to the ambient temperature to obtain the actual body temperature of the organism. By providing the processing unit, the obtained actual body temperature of the organism is more accurate, thus facilitating accurately judging the health status of the organism. In addition, the temperature measurement device is economical in consumables, easy to implement and convenient to use.

It can be understood that, the foregoing implementations are merely exemplary implementations used for explaining the principle of the present invention, rather than limiting the present invention. Those of ordinary skill in the art may make various variations and improvements without departing from the spirit and essence of the present invention, and these variations and improvements should be encompassed in the protection scope of the present invention.

## Claims

1. A temperature measurement device, comprising:
a first sensor, used for collecting a body surface temperature of an organism;
a second sensor, used for collecting an ambient temperature; and
a processing unit, which is electrically connected with the first sensor and the second sensor, respectively, and is used for performing data processing on the body surface temperature according to the ambient temperature to obtain an actual body temperature of the organism.

2. The temperature measurement device of claim 1, wherein the processing unit comprises a compensation module, which is used for compensating the body surface temperature with a compensation value, the compensation value is a deviation of the ambient temperature from an ambient normal temperature, and the ambient normal temperature is a standard temperature of an environment where the organism is located.

3. The temperature measurement device of claim 2, wherein the processing unit further comprises an analog/digital conversion module, which is electrically connected with the first sensor, the second sensor and the compensation module, and is used for respectively converting the body surface temperature and the ambient temperature from analog quantities into digital quantities and transmitting the digital quantities of the body surface temperature and the ambient temperature to the compensation module.

4. The temperature measurement device of claim 2, wherein the processing unit further comprises an averaging module, which is electrically connected with the compensation module and is used for averaging two or more compensated body surface temperatures to obtain an average value serving as the actual body temperature of the organism.

5. The temperature measurement device of claim 2, wherein the processing unit further comprises a correction module, which is electrically connected with the compensation module and is used for correcting the compensated body surface temperature with a correction value, and the correction value is an empirical value obtained through a plurality of tests and represents a deviation of an actual body surface temperature from a true body temperature of the organism.

6. The temperature measurement device of claim 5, wherein the processing unit further comprises an averaging module, which is electrically connected with the correction module and is used for averaging two or more compensated and corrected body surface temperatures to obtain an average value serving as the actual body temperature of the organism.

7. The temperature measurement device of claim 1, further comprising a storage unit, which is electrically connected with the processing unit, and is used for storing data in data processing.

8. The temperature measurement device of any one of claims 1 to 7, further comprising an annular belt, which is worn on the organism, wherein the first sensor is arranged at an inner side of the annular belt facing a body surface of the organism, and the second sensor is arranged at an outer side of the annular belt away from the body surface of the organism.

9. The temperature measurement device of claim 8, wherein a distance from the first sensor to the body surface of the organism is in the range of 0 to 2mm, and a distance from the second sensor to the body surface of the organism is in the range of 5mm to 8mm.

10. The temperature measurement device of claim 1, wherein the body surface temperature comprises a body surface temperature at wrist of the organism, a body surface temperature at neck of the organism or a body surface temperature at ankle of the organism.

11. The temperature measurement device of claim 1, wherein the processing unit comprises a single chip microcomputer, a DSP or an FPGA, and the first sensor and the second sensor respectively transmit the body surface temperature and the ambient temperature to the processing unit through a communication protocol.

12. A temperature measurement method, comprising steps of:
S1: collecting a body surface temperature of an organism, and collecting an ambient temperature; and
S2: performing data processing on the body surface temperature according to the ambient temperature to obtain an actual body temperature of the organism.

13. The temperature measurement method of claim 12, wherein step S2 comprises steps of:
S21: compensating the body surface temperature with a compensation value, which is a deviation of the ambient temperature from an ambient normal temperature, wherein the ambient normal temperature is a standard temperature of an environment where the organism is located; and
S22: correcting the compensated body surface temperature with a correction value, which is an empirical value obtained through a plurality of tests and represents a deviation of an actual body surface temperature from a true body temperature of the organism.

14. The temperature measurement method of claim 13, wherein step S2 further comprises a step of:
S23: repeatedly performing step S1, step S21 and step S22 for twice or more times, and then averaging the obtained two or more compensated and corrected body surface temperatures.

15. The temperature measurement method of claim 13 or 14, wherein before step S21, the method further comprises: converting the body surface temperature and the ambient temperature from analog quantities into digital quantities, respectively.
